# EUROPEAN PATENT SPECIFICATION

(11) **EP 2 646 424 B1**
(45) Date of publication and mention of the grant of the patent: **30.09.2015**
(21) Application number: 11844252.4
(22) Date of filing: 23.11.2011
(51) Int. Cl.: C07D 295/084

(54) **PROCESSES FOR THE PREPARATION OF ENAMINES**
VERFAHREN ZUR HERSTELLUNG VON ENAMINEN
PROCESSUS DE PRÉPARATION D'ÉNAMINES

(30) Priority: 03.12.2010 US 419277 P
(43) Date of publication of application: 09.10.2013
(73) Proprietor: Dow AgroSciences LLC, Indianapolis, IN 46268 (US)
(72) Inventor: BLAND, Douglas C., Midland, MI 48642 (US); TOYZAN, Todd William, Freeland, MI 48623 (US); LENG, Ronald B., Midland, MI 48640 (US); MCCONNELL, James R., Midland, MI 48640 (US)
(74) Representative: f & e patent
(86) International application number: PCT/US2011/061986
(87) International publication number: WO 2012/074862

(56) References cited:
- WO-A1-2010/002577
- WO-A2-2009/007460
- US-A- 3 074 940
- US-A- 3 530 120
- CARLSON ET AL.: 'Improved Titanium Tetrachloride Procedure for Enamine Synthesis. Scope of the Reaction.' ACTA CHEMICA SCANDINAVICA B vol. 38, 1984, pages 49 - 53, XP055117483

## Description

### FIELD OF THE INVENTION

The invention disclosed in this document is related to the field of processes for the preparation of enamines.

### BACKGROUND OF THE INVENTION

Enamines are very useful molecules. They have been used in a wide variety of reactions such as, for example, electrophilic substitution and addition, oxidation and reduction, and cycloaddition (J. Kang, Y. R. Cho, and J. H. Lee, Bull. Korean Chem Soc. Vol. 13, No.2, 1992).

An early method for preparing enamines involved the condensation of aldehydes and ketones with secondary amines (C. Mannich and H. Davidsen, Ber., 69, 2106 (1936). Mannich and Davidsen discovered that the condensation reaction of an aldehyde with a secondary amine could be conducted at temperatures near 0 °C in the presence of potassium carbonate (K₂CO₃), but however, the condensation reaction of a ketone with a secondary amine required calcium oxide (CaO) and elevated temperatures. Later, Herr and Heyl discovered that this type of condensation reaction could be improved by removing water (H₂O) during an azeotropic distillation with benzene (M.E. Herr and F. W. Heyl, J. Am. Chem. Soc., 74, 3627 (1952); F. W. Heyl and M.E. Herr , J. Am. Chem. Soc., 75, 1918 (1953); M.E. Herr and F. W. Heyl, J. Am. Chem. Soc., 75, 5927 (1953); F. W. Heyl and M.E. Herr , J. Am. Chem. Soc., 77, 488 (1955)). Since these publications a number of modifications have been disclosed. Usually, these modifications are based on using dehydration reagents such as K₂CO₃, CaO, *p*-toluenesulfonic acid (TsOH), boron trifluoride diethyl etherate (BF₃-OEt₂), acetic acid (AcOH), magnesium sulfate (MgSO₄), calcium hydride (CaH₂), titanium tetrachloride (TiCl₄), and molecular sieves (see J. Kang above). Other modifications deal with chemically converting water to something else during the condensation reaction (see J. Kang above). An extensive summary of the vast number of methods to prepare enamines is discussed in "ENAMINES, Synthesis, Structure, and Reactions, 2nd Edition, Edited by A. G. Cook, Chap. 2, (1988). Specific examples of processes to prepare enamines can be found in the following:
U.S. Patent 3,074,940 which discloses that certain aldehydes form azeotropes with water which can be used to remove the reaction water formed during certain enamine condensation reactions;
U.S. Patent 3,530,120 which discloses conducting certain enamine condensation reactions in an inert atmosphere with certain arsine molecules;
U.S. Patent 5,247,091 which discloses conducting certain enamine condensation reactions in an aqueous media;
S. Kaiser, S. P. Smidt, and A. Pfaltz, Angew. Int. Ed. 2006, 45, 5194-5197-See Supporting information pages 10-11; and
WO 2009/007460 A2, see page 13, example 1.a.

Enamines such as 1-(3-methylthiobut-1-enyl)pyrrolidine are useful intermediates for the preparation of certain new insecticides (see, for example, U.S. Patent Publications 2005/0228027 and 2007/0203191). Current known processes to make such thioenamines are not efficient in producing such enamines due to a variety of reasons -- there are problems in preventing thermal degradation of the thioenamine, and while using potassium carbonate is an effective desiccant, it is problematic to filter such desiccant during larger than lab-scale production. Thus, a process is needed to remove water during these types of condensation reactions without using solid desiccants, or using temperature conditions that promote the thermal degradation of such enamines.

### DETAILED DESCRIPTION OF THE INVENTION

In general, the processes disclosed in this document can be illustrated as in Scheme 1.

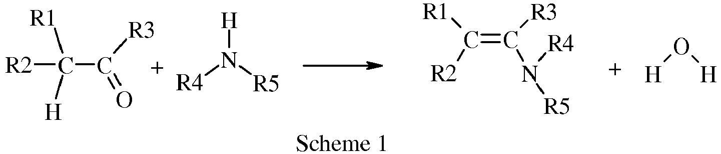

In general, the invention is a process comprising:
(A) contacting a first mixture with a second mixture in a reaction zone,
   (1) wherein said first mixture comprises an amine having the following formula 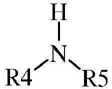 wherein R4 and R5 are each independently selected from C₁-C₈ alkyl, C₃-C₈ cycloalkyl, C₂-C₈ alkoxyalkyl, C₇-C₁₂ arylalkyl, C₂-C₈ alkylaminoalkyl, aryl, and heteroaryl, or R4 and R5 taken together with N represent a 5- or 6-membered saturated or unsaturated ring, and
   (2) wherein said second mixture comprises a non-polar-high-boiling-point-solvent and a carbonyl (i.e. an aldehyde or a ketone) having the following formula 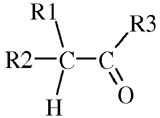
      (a) wherein R1 and R2 is each independently selected from C₁-C₈ alkyl, C₃-C₈ cycloalkyl, C₂-C₈ alkoxyalkyl, C₇-C₁₂ arylalkyl, C₂-C₈ alkylaminoalkyl, aryl, and heteroaryl, each of which is independently substituted with one or more S-R6 wherein each R6 is independently selected from C₁-C₈ alkyl, C₃-C₈ cycloalkyl, C₂-C₈ alkoxyalkyl, C₇-C₁₂ arylalkyl, C₂-C₈ alkylaminoalkyl, aryl, and heteroaryl, and
      (b) wherein R3 is selected from H, C₁-C₈ alkyl, C₃-C₈ cycloalkyl, C₂-C₈ alkoxyalkyl, C₇-C₁₂ arylalkyl, C₂-C₈ alkylaminoalkyl, aryl, and heteroaryl;
(B) reacting in said reaction zone said amine and said carbonyl to produce an enamine and H₂O, wherein said reacting is conducted under distillation conditions comprising
   (1) a pressure from about 100 Pascals (Pa) to about 120,000 Pa, and
   (2) a temperature below about, but preferably below, the thermal decomposition temperature of said enamine during said reacting; and
(C) removing a vapor phase from said reaction zone wherein said vapor phase comprises said non-polar-high-boiling-point-solvent and H₂O,
wherein the ratio of
(the amount of first mixture added to said reaction zone) :
(the amount of vapor phase removed from said reaction zone)
is from about
(1 part first mixture added) :
(1 part vapor phase removed)
to about (1 part first mixture added) :
(20 parts vapor phase removed).

In general said contacting can be done in any manner, however, it is preferred if said first mixture is contacted with said second mixture in said reaction zone such that said contacting takes place at or below the surface of said second mixture.

Approximately equimolar quantities of said amine and said carbonyl can be used in the process, although excesses of one or the other may be employed. The molar ratio of amine to carbonyl can be from about 0.9 to about 1.2, however, a slight molar excess of amine to carbonyl is preferred, such as, for example, a molar ratio greater than 1 but less than about 1.1.

The reaction is conducted in the presence of a non-polar-high-boiling-point-solvent such as, hydrocarbon solvents, most preferably aromatic hydrocarbon solvents such as, for example, benzene, toluene, or xylene. Currently, toluene is a preferred solvent.

In another embodiment of this invention, said reacting is conducted under distillation conditions comprising a temperature that keeps the majority, if not all, of said carbonyl, which has not reacted, preferably in said second mixture and not in said vapor phase. It is preferable to keep the carbonyl in the second mixture so that it can react with the amine and not form a water-aldehyde azeotrope. For example, if butyraldehyde is used, a desirable temperature range would be about 60 °C to about 80 °C around one atmosphere of pressure.

In another embodiment of this invention said reacting is conducted under distillation conditions comprising a pressure from about 1000 Pa to about 60,000 Pa and a temperature from about 10 °C to about 80 °C.

In another embodiment of this invention said reacting is conducted under distillation conditions comprising a pressure from about 2500 Pa to about 30,000 Pa and a temperature from about 20 °C to about 70 °C.

In another embodiment of this invention said reacting is conducted under distillation conditions comprising a pressure from about 5000 Pa to about 15,000 Pa and a temperature from about 25 °C to about 65 °C. In another embodiment of this invention when producing 1-(3-methylsulfanyl-but-1-enyl)-pyrrolidine a temperature below about the thermal decomposition temperature of 1-(3-methylsulfanyl-but-1-enyl)-pyrrolidine during said reacting is preferred.

It is preferred in such processes that the condensation reaction be conducted under azeotropic conditions so that as much water can be removed as desired. It is also preferred if no desiccants be used to remove water.

In another embodiment of this invention, R1 and R2 are independently C₁-C₈ alkyl, C₃-C₈ cycloalkyl, each of which is independently substituted with one or more S-R6 wherein each R6 is independently selected from C₁-C₈ alkyl.

In another embodiment of this invention, R3 is H.

In another embodiment of this invention, wherein R4 and R5 are each independently selected from C₁-C₈ alkyl and C₃-C₈ cycloalkyl. In another embodiment of this invention R4 and R5 taken together with N represent a 5- or 6-membered saturated or unsaturated ring.

In another embodiment of this invention, said first mixture comprises pyrrolidine and said second mixture comprises 3-methylsulfanyl-butyraldehyde. In another embodiment of this invention, said enamine is 1-(3-methylsulfanyl-but-1-enyl)-pyrrolidine.

In another embodiment of this invention, the first mixture and second mixture can be contacted in the reaction zone simultaneously as they are added.

In another embodiment of the invention, the ratio of
(the amount of first mixture added to said reaction zone) :
(the amount of vapor phase removed from said reaction zone) is from about
(1 part first mixture added) :
(2 parts vapor phase removed)
to about (1 part first mixture added) :
(15 parts vapor phase removed).

In another embodiment of the invention, the ratio of
(the amount of first mixture added to said reaction zone) :
(the amount of vapor phase removed from said reaction zone) is from about
(1 part first mixture added) :
(3 parts vapor phase removed)
to about (1 part first mixture added) :
(10 parts vapor phase removed).

### EXAMPLES

The examples are for illustration purposes only.

### 1. Preparation of 3-methylthiobutanal (1) from crotonaldehyde.

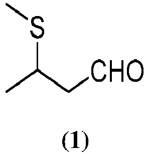

To a three Liter (L) 3-neck round bottom flask equipped with magnetic stirring, temperature probe, addition funnel, distillation head, padded with nitrogen, and vented to a bleach scrubber was charged with 100 mL toluene followed by 84 g (1.39 mol) of glacial acetic acid followed by 61 g (0.86 mol) of crotonaldehyde. Another 100 mL of toluene was used as solvent rinses during the addition of acetic acid and crotonaldehyde. The reaction mixture was cooled in an ice-water bath and then 500 g (0.906 mol) of a 12.7 wt% aqueous sodium methyl mercaptide solution was added via addition funnel over a 67 minutes (min) period. The internal reaction temperature rose from 2 °C to 13 °C during addition of the mercaptide solution, and the reaction pH tested around ∼7 using pH test strips. The ice-water bath was removed and the reaction was heated to 50 °C for 10 hours (h). At this time, gas chromatographic (GC) analysis indicated about -0.8% (relative area) for the crotonaldehyde starting material. The reaction mixture was then transferred to a 2-L separatory funnel and the mixture was diluted with another 400 mL of toluene. The bottom aqueous layer was drained and discarded. The remaining organic layer was washed with 300 mL of fresh water. The bottom aqueous wash layer was discarded and the remaining organic layer was transferred back to the reaction vessel. The reaction mixture was then azeotropically dried at a temperature range of 19 °C to 22 °C and a vacuum of -5300 Pa Hg for about 40 min. The collected distillate contained mostly toluene and about 0.2% of 3-methylthiobutanal. After completing the distillation, the remaining reaction bottoms in the pot were isolated to give 536 g of 3-methylthiobutanal in toluene as a light yellow solution. GC assay analysis of this mixture (using dipropyl phthalate as internal standard) indicated a 17.6 wt% solution of 3-methylthiobutanal (1) in toluene and a 93% in-pot yield.

### 2. Preparation of 3-methylthiobutanal (1) from crotonaldehyde .

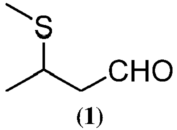

To a 500 mL three neck round bottom flask was charged sequentially 25.00 g (0.35 mol) of 99% crotonaldehyde, then 28.03 g (0.47 mol) of glacial acetic acid, and finally 57.26 g (0.62 mol) of toluene. The reaction mixture was stirred magnetically and cooled in an ice-water bath. Once the internal reaction temperature reached 2 °C, 143.79 g (0.431 mol) of a 21 wt% aqueous sodium methylmercaptide solution was continuously added via addition funnel over a 56 min period and the internal reaction temperature rose from 2 °C to 10 °C during the addition this time. The pH was measured around 7.0 using a test strip paper. The ice-water bath was removed and the reaction was heated at 60 °C for 24 h at which time the reaction mixture was allowed to cool. The reaction mixture phases were separated. The bottom aqueous phase (147.95 g) was discarded into the waste stream. The top organic phase (97.6 g) was isolated. GC assay analysis of this mixture (using dipropyl phthalate as internal standard) indicated a 37.5 wt% solution of 3-methylthiobutanal **(1)** in toluene and a 88% in-pot yield.

### 3. Preparation of 1-(3-methylsulfanylbut-1-enyl)pyrrolidine (2).

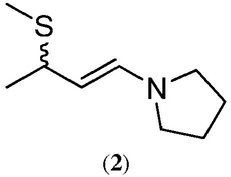

To a 500 mL three-neck round bottom flask fitted with a fractional distillation head was charged the 96.55 g (0.31 mol) of a 37.5 wt% 3-methylthiobutanal in toluene solution (from Example 2) followed by an additional 276 g (3.0 mol) of fresh toluene. The reaction mixture was heated to 35 °C and the system was put on total reflux under a reduced pressure of ∼9300-10,600 Pa. The mixture was stirred for 45 min at total reflux and then 15.5 g of distillate was collected overhead for a 22.0 min period while the pot temperature was about 39 °C. An additional 16.5 g of distillate was collected over a 12 min period while the pot temperature was about 46 °C. After the second fraction was collected, 21.8 g (0.31 mol) of pyrrolidine was continuously added subsurface to the reaction mixture over a 55.0 min period. During the pyrrolidine addition, the following distillation ranges were observed:

| | |
|---|---|
| Pot temperature: | 35-47 °C |
| Overheads temperature | 30-47 °C |
| Pressure | about 9300-10,600 Pa |

At the end of the pyrrolidine addition, the subsurface line was rinsed with about 0.86 g of toluene. The distillation was continued an additional 47 minutes taking lights overhead. The vacuum was relieved by purging the system with nitrogen, and then the mixture was cooled to ambient temperature. A total of 146.21 g of distillate was collected. A total of 186.82 g of distillation bottoms was collected and analyzed for product yield. ¹H NMR spectroscopic assay of this product mixture (using benzyl acetate as an internal standard and CDCl₃ as solvent) indicated a 24.6 wt% solution of 1-(3-methylsulfanylbut-1-enyl)pyrrolidine (2) in toluene and an 87% in-pot yield.

### 4. Preparation of 1-(3-methylsulfanylbut-1-enyl)pyrrolidine (2).

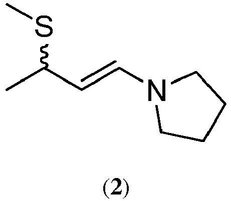

To a 700 mL three-neck jacketed reactor fitted with a fractional distillation head was charged the 17.00 g (0.326 mol) of a 22.7 wt% 3-methylthiobutanal in toluene solution followed by an additional 284 g (9.44 mol) of fresh toluene. The reaction mixture was heated to 45 °C and placed under -10,600 Pa reduced pressure, and then 24.63 g (0.343 mol) of pyrrolidine was continuously added subsurface to the reaction mixture over a 15.0 min period. During the pyrrolidine addition, the following distillation ranges were observed:

| | |
|---|---|
| Pot temperature: | 41-45 °C |
| Overheads temperature | 38-40 °C |
| Pressure | about 10,600 Pa |

At the end of the pyrrolidine addition, the subsurface line was rinsed with about 0.86 g of toluene. The distillation was continued an additional 28 min taking lights overhead. The vacuum was relieved by purging the system with nitrogen, and then the mixture was cooled to ambient temperature. A total of 248.25 g of distillate was collected. A total of 192.70 g of distillation bottoms was collected and analyzed for product yield. ¹H NMR spectroscopic assay of this product mixture (using benzyl acetate as an internal standard and CDCl₃ as solvent) indicated a 19.3 wt% solution of 1-(3-methylsulfanylbut-1-enyl)pyrrolidine (2) in toluene and an 86% in-pot yield.

## Claims

1. A process comprising:
(A) contacting a first mixture with a second mixture in a reaction zone,
(1) wherein said first mixture comprises an amine having the following formula 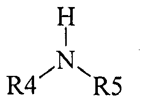 wherein R4 and R5 are each independently selected from C₁-C₈ alkyl, C₃-C₈ cycloalkyl, C₂-C₈ alkoxyalkyl, C₇-C₁₂ arylalkyl, C₂-C₈ alkylaminoalkyl, aryl, and heteroaryl, or R4 and R5 taken together with N represent a 5- or 6-membered saturated or unsaturated ring, and
(2) wherein said second mixture comprises a non-polar-high-boiling-point-solvent and a carbonyl (i.e. an aldehyde or a ketone) having the following formula 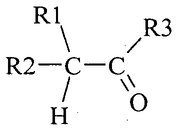
(a) wherein R1 and R2 is each independently selected from C₁-C₈ alkyl, C₃-C₈ cycloalkyl, C₂-C₈ alkoxyalkyl, C₇-C₁₂ arylalkyl, C₂-C₈ alkylaminoalkyl, aryl, and heteroaryl, each of which is independently substituted with one or more S-R6 wherein each R6 is independently selected from C₁-C₈ alkyl, C₃-C₈ cycloalkyl, C₂-C₈ alkoxyalkyl, C₇-C₁₂ arylalkyl, C₂-C₈ alkylaminoalkyl, aryl, and heteroaryl, and
(b) wherein R3 is selected from H, C₁-C₈ alkyl, C₃-C₈ cycloalkyl, C₂-C₈ alkoxyalkyl, C₇-C₁₂ arylalkyl, C₂-C₈ alkylaminoalkyl, aryl, and heteroaryl;
(B) reacting in said reaction zone said amine and said carbonyl to produce an enamine and H₂O, wherein said reacting is conducted under distillation conditions comprising
(1) a pressure from about 100 Pascals (Pa) to about 120,000 Pa, and
(2) a temperature below about, but preferably below, the thermal decomposition temperature of said enamine during said reacting; and
(C) removing a vapor phase from said reaction zone wherein said vapor phase comprises said non-polar-high-boiling-point-solvent and H₂O,
wherein the ratio of
(the amount of first mixture added to said reaction zone) :
(the amount of vapor phase removed from said reaction zone) is from about
(1 part first mixture added) :
(1 part vapor phase removed)
to about
(1 part first mixture added) :
(20 parts vapor phase removed).

2. A process according to claim 1 wherein said first mixture is contacted with said second mixture in said reaction zone such that said contacting takes place at or below the surface of said second mixture.

3. A process according to claim 1 wherein the molar ratio of amine to carbonyl, used during the reaction is greater than 1 but less than about 1.1.

4. A process according to claim 1 wherein said non-polar-high-boiling-point-solvent is toluene.

5. A process according to claim 1 wherein said reacting is conducted under distillation conditions comprising a temperature that keeps the majority, if not all, of said carbonyl, which has not reacted, in said second mixture and not in said vapor phase.

6. A process according to claim 1 wherein said reacting is conducted under distillation conditions comprising a pressure from about 1000 Pa to about 60,000 Pa and a temperature from about 10 °C to about 80 °C, preferably a pressure from about 2500 Pa to about 30,000 Pa and a temperature from about 20 °C to about 70 °C, more preferably a pressure from about 5000 Pa to about 15,000 Pa and a temperature from about 25 °C to about 65 °C.

7. A process according to claim 1 wherein said reacting is conducted under azeotropic conditions.

8. A process according to claim 1 wherein R1 and R2 are independently C₁-C₈ alkyl, C₃-C₈ cycloalkyl, each of which is independently substituted with one or more S-R6 wherein each R6 is independently selected from C₁-C₈ alkyl.

9. A process according to claim 1 wherein R3 is H.

10. A process according to claim 1 wherein R4 and R5 are each independently selected from C₁-C₈ alkyl, and C₃-C₈ cycloalkyl.

11. A process according to claim 1 wherein R4 and R5 taken together with N represent a 5- or 6-membered saturated or unsaturated ring.

12. A process according to claim 1 wherein the ratio of
(the amount of first mixture added to said reaction zone) :
(the amount of vapor phase removed from said reaction zone) is from about
(1 part first mixture added) :
(2 parts vapor phase removed)
to about
(1 part first mixture added) :
(15 parts vapor phase removed),
preferably from about
(1 part first mixture added) :
(3 parts vapor phase removed)
to about
(1 part first mixture added) :
(10 parts vapor phase removed).

13. A process comprising:
(A) contacting a first mixture with a second mixture in a reaction zone
(1) wherein said first mixture comprises pyrrolidine, and
(2) wherein said second mixture comprises 3-methylsulfanyl-butyraldehyde and toluene;
(B) reacting in said reaction zone said pyrrolidine and said 3-methylsulfanyl-butyraldehyde to produce 1-(3-methylsulfanyl-but-1-enyl)-pyrrolidine and H₂O, wherein said reacting is conducted under distillation conditions comprising
(1) a pressure from about 5000 Pa to about 15000 Pa, and
(2) a temperature from about 25 °C to about 65 °C; and
(C) removing a vapor phase comprising toluene and H₂O and essentially no 3-methylsulfanyl-butyraldehyde,
wherein the ratio of
(the amount of first mixture added) : (the vapor phase removed) is from about
(1 part first mixture added) : (3 parts vapor phase removed) to about
(1 part first mixture added) : (10 parts vapor phase removed).

## Patentansprüche

1. Ein Verfahren umfassend:
(A) In-Kontakt-Bringen einer ersten Mischung mit einer zweiten Mischung in einer Reaktionszone,
(1) wobei diese erste Mischung ein Amin mit der nachfolgenden Formel enthält 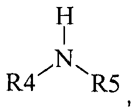 wobei R4 und R5 jeweils unabhängig voneinander aus C₁-C₈-Alkyl, C₃-C₈-Cycloalkyl, C₂-C₈-Alkoxyalkyl, C₇-C₁₂-Arylalkyl, C₂-C₈-Alkylaminoalkyl, Aryl und Heteroaryl ausgewählt sind oder R4 und R5 zusammengenommen mit N einen 5- oder 6-gliedrigen gesättigten oder ungesättigten Ring darstellen und
(2) wobei die zweite Mischung ein nichtpolares Lösungsmittel mit hohem Siedepunkt und ein Carbonyl (das heißt ein Aldehyd oder ein Keton) mit der nachfolgenden Formel enthält 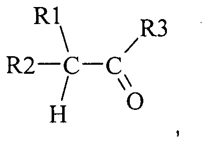
(a) wobei R1 und R2 jeweils unabhängig voneinander aus C₁-C₈-Alkyl, C₃-C₈-Cycloalkyl, C₂-C₈-Alkoxyalkyl, C₇-C₁₂-Arylalkyl, C₂-C₈-Alkylaminoalkyl, Aryl und Heteroaryl ausgewählt sind, von denen jedes unab-hängig voneinander mit einem oder mehreren S-R6 substituiert ist, wobei jedes R6 unabhängig voneinander aus C₁-C₈-Alkyl, C₃-C₈-Cycloalkyl, C₂-C₈-Alk-oxyalkyl, C₇-C₁₂-Arylalkyl, C₂-C₈-Alkylaminoalkyl, Aryl und Heteroaryl ausgewählt ist, ausgewählt sind, und
(b) wobei R3 aus H, C₁-C₈-Alkyl, C₃-C₈-Cycloalkyl, C₂-C₈-Alkoxyalkyl, C₇-C₁₂-Arylalkyl, C₂-C₈-Alkylaminoalkyl, Aryl und Heteroaryl ausgewählt ist;
(B) Umsetzen des Amins und des Carbonyls in der Reaktionszone, um ein Enamin und H₂O zu erzeugen, wobei dieses Umsetzen unter Destillationsbedingungen durchgeführt wird, welche umfassen:
(1) einen Druck von etwa 100 Pascal (Pa) bis etwa 120.000 Pa und
(2) eine Temperatur ungefähr unterhalb, aber vorzugsweise unterhalb der thermischen Zersetzungstemperatur des Enamins während dieser Umsetzung, und
(C) Entfernen einer Dampfphase aus der Reaktionszone, wobei diese Dampfphase das nichtpolare Lösungsmittel mit hohem Siedepunkt und H₂O umfasst,
wobei das Verhältnis von
(der Menge der in die Reaktionszone hinzugefügten ersten Mischung) : (der Menge der aus dieser Reaktionszone entfernten Dampfphase)
von etwa
(1 Teil hinzugefügter erster Mischung) :
(1 Teil entfernter Dampfphase)
bis etwa
(1 Teil hinzugefügter erster Mischung) :
(20 Teile entfernter Dampfphase) beträgt.

2. Ein Verfahren gemäß Anspruch 1, wobei die erste Mischung mit der zweiten Mischung in der Reaktionszone in einer solchen Weise in Kontakt gebracht wird, dass das In-Kontakt-Bringen an oder unterhalb der Oberfläche der zweiten Mischung stattfindet.

3. Ein Verfahren gemäß Anspruch 1, wobei das molare Verhältnis von Amin zu Carbonyl, welches während der Reaktion verwendet wird, größer als 1, aber kleiner als etwa 1,1 ist.

4. Ein Verfahren gemäß Anspruch 1, wobei das nichtpolare Lösungsmittel mit hohem Siedepunkt Toluol ist.

5. Ein Verfahren gemäß Anspruch 1, wobei die Umsetzung unter Destillationsbedingungen durchgeführt wird, die eine Temperatur, welche den Großteil des Carbonyls, das nicht umgesetzt wurde, wenn nicht alles, in der zweiten Mischung und nicht in der Dampfphase hält, umfassen.

6. Ein Verfahren gemäß Anspruch 1, wobei die Umsetzung unter Destillationsbedingungen durchgeführt wird, die einen Druck von etwa 1.000 Pa bis etwa 60.000 Pa und eine Temperatur von etwa 10 °C bis etwa 80 °C umfassen, vorzugsweise einen Druck von etwa 2.500 Pa bis etwa 30.000 Pa und eine Temperatur von etwa 20 °C bis etwa 70 °C, weiterhin bevorzugt einen Druck von etwa 5.000 Pa bis etwa 15.000 Pa und eine Temperatur von etwa 25 °C bis etwa 65 °C.

7. Ein Verfahren gemäß Anspruch 1, wobei die Umsetzung unter azeotropen Bedingungen durchgeführt wird.

8. Ein Verfahren gemäß Anspruch 1, wobei R1 und R2 unabhängig voneinander C₁-C₈-Alkyl oder C₃-C₈-Cycloalkyl sind, von denen jedes unabhängig voneinander mit einem oder mehreren S-R6 substituiert ist, wobei jedes R6 unabhängig voneinander aus C₁-C₈-Alkyl ausgewählt ist.

9. Ein Verfahren gemäß Anspruch 1, wobei R3 gleich H ist.

10. Ein Verfahren gemäß Anspruch 1, wobei R4 und R5 jeweils unabhängig voneinander aus C₁-C₈-Alkyl und C₃-C₈-Cycloalkyl ausgewählt sind.

11. Ein Verfahren gemäß Anspruch 1, wobei R4 und R5 zusammengenommen mit N einen 5- oder 6-gliedrigen gesättigten oder ungesättigten Ring darstellen.

12. Ein Verfahren gemäß Anspruch 1, wobei das Verhältnis von
(der Menge der in die Reaktionszone hinzugefügten ersten Mischung) : (der Menge der aus dieser Reaktionszone entfernten Dampfphase)
von etwa
(1 Teil hinzugefügter erster Mischung) :
(2 Teile entfernter Dampfphase)
bis etwa
(1 Teil hinzugefügter erster Mischung) :
(15 Teile entfernter Dampfphase),
vorzugsweise von etwa
(1 Teil hinzugefügter erster Mischung) :
(3 Teile entfernter Dampfphase)
bis etwa
(1 Teil hinzugefügter erster Mischung) :
(10 Teile entfernter Dampfphase)
beträgt.

13. Ein Verfahren umfassend:
(A) In-Kontakt-Bringen einer ersten Mischung mit einer zweiten Mischung in einer Reaktionszone,
(1) wobei die erste Mischung Pyrrolidin umfasst, und
(2) die zweite Mischung 3-Methylsulfanyl-butyraldehyd und Toluol umfasst;
(B) Umsetzen des Pyrrolidins und des 3-Methylsulfanylbutyraldehyds in dieser Reaktionszone, um 1-(3-Methylsulfanylbut-1-enyl)pyrrolidin und H₂O zu erzeugen, wobei die Umsetzung unter Destillations-bedingungen durchgeführt wird, die umfassen:
(1) einen Druck von etwa 5.000 Pa bis etwa 15.000 Pa, und
(2) eine Temperatur von etwa 25 °C bis etwa 65 °C, und
(C) Entfernen einer Dampfphase umfassend Toluol und H₂O und im Wesentlichen kein 3-Methylsulfanylbutyraldehyd,
wobei das Verhältnis von
(der Menge der hinzugefügten ersten Mischung) : (der entfernten Dampfphase)
von etwa
(1 Teil hinzugefügte erste Mischung) : (3 Teile entfernte Dampfphase)
bis etwa
(1 Teil hinzugefügte erste Mischung) : (10 Teile entfernte
Dampfphase)
beträgt.

## Revendications

1. Procédé comprenant :
(A) la mise en contact d'un premier mélange avec un second mélange dans une zone de réaction,
(1) le premier mélange comprenant une amine correspondant à la formule suivante 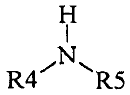 dans laquelle R4 et R5 sont choisis chacun indépendamment parmi des groupes alkyle en C₁-C₈, cycloalkyle en C₃-C₈, alcoxyalkyle en C₂-C₈, arylalkyle en C₇-C₁₂, alkylaminoalkyle en C₂-C₈, aryle et hétéroaryle, ou R4 et R5 pris ensemble avec N représentent un cycle saturé ou insaturé à 5 ou 6 chaînons, et
(2) le second mélange comprenant un solvant non polaire à haut point d'ébullition et un composé carbonylé (c'est-à-dire un aldéhyde ou une cétone) correspondant à la formule suivante 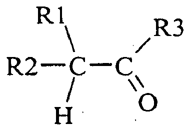
(a) dans laquelle R1 et R2 sont choisis chacun indépendamment parmi des groupes alkyle en C₁-C₈, cycloalkyle en C₃-C₈, alcoxyalkyle en C₂-C₈, arylalkyle en C₇-C₁₂, alkylaminoalkyle en C₂-C₈, aryle et hétéroaryle, chacun d'eux portant indépendamment un ou plusieurs substituants S-R6, chaque radical R6 étant choisi indépendamment parmi des groupes alkyle en C₁-C₈, cycloalkyle en C₃-C₈, alcoxyalkyle en C₂-C₈, arylalkyle en C₇-C₁₂, alkylaminoalkyle en C₂-C₈, aryle et hétéroaryle ; et
(b) dans laquelle R3 est choisi parmi H, des groupes alkyle en C₁-C₈, cycloalkyle en C₃-C₈, alcoxyalkyle en C₂-C₈, arylalkyle en C₇-C₁₂, alkylaminoalkyle en C₂-C₈, aryle et hétéroaryle,
(B) la mise en réaction dans ladite zone de réaction de ladite amine et dudit composé carbonyle, pour l'obtention d'une énamine et H₂O, ladite réaction étant effectuée dans des conditions de distillation comprenant
(1) une pression d'environ 100 pascals (Pa) à environ 120 000 Pa, et
(2) une température inférieure à environ, mais de préférence inférieure à la température de décomposition thermique de ladite énamine au cours de ladite réaction ; et
(C) l'évacuation d'une phase vapeur de ladite zone de réaction, ladite phase vapeur comprenant ledit solvant non polaire à haut point d'ébullition et H₂O,
dans lequel le rapport de
(la quantité du premier mélange introduit dans la zone de réaction) :
(la quantité de phase vapeur évacuée de ladite zone de réaction) vaut d'environ
(1 partie du premier mélange introduit) :
(1 partie de phase vapeur évacuée)
à environ
(1 partie du premier mélange introduit) :
(20 parties de phase vapeur évacuée).

2. Procédé selon la revendication 1, dans lequel on met ledit premier mélange en contact avec ledit second mélange dans ladite zone de réaction de manière que ladite mise en contact ait lieu à ou au-dessous de la surface dudit second mélange.

3. Procédé selon la revendication 1, dans lequel le rapport molaire de l'amine au composé carbonylé utilisé au cours de la réaction est supérieur à 1 mais inférieur à environ 1,1.

4. Procédé selon la revendication 1, dans lequel ledit solvant non polaire à haut point d'ébullition est le toluène.

5. Procédé selon la revendication 1, dans lequel ladite réaction est effectuée dans des conditions de distillation comprenant une température qui maintient la majeure partie, sinon la totalité, dudit composé carbonylé, qui n'a pas réagi, dans ledit second mélange et non dans ladite phase vapeur.

6. Procédé selon la revendication 1, dans lequel ladite réaction est effectuée dans des conditions de distillation comprenant une pression d'environ 1 000 Pa à environ 60 000 Pa et une température d'environ 10 °C à environ 80 °C, de préférence une pression d'environ 2 500 Pa à environ 30 000 Pa et une température d'environ 20 °C à environ 70 °C, encore mieux une pression d'environ 5 000 Pa à environ 15 000 Pa et une température d'environ 25 °C à environ 65 °C.

7. Procédé selon la revendication 1, dans lequel ladite réaction est effectuée dans des conditions azéotropiques.

8. Procédé selon la revendication 1, dans lequel R1 et R2 représentent indépendamment un groupe alkyle en C₁-C₈, cycloalkyle en C₃-C₈, dont chacun porte indépendamment un ou plusieurs substituants S-R6, chaque radical R6 étant indépendamment choisi parmi des groupes alkyle en C₁-C₈.

9. Procédé selon la revendication 1, dans lequel R3 est H.

10. Procédé selon la revendication 1, dans lequel R4 et R5 sont choisis chacun indépendamment parmi des groupes alkyle en C₁-C₈ et cycloalkyle en C₃-C₈.

11. Procédé selon la revendication 1, dans lequel R4 et R5 pris ensemble avec N représentent un cycle saturé ou insaturé à 5 ou 6 chaînons.

12. Procédé selon la revendication 1, dans lequel le rapport de
(la quantité du premier mélange introduit dans la zone de réaction) :
(la quantité de phase vapeur évacuée de ladite zone de réaction)
vaut d'environ
(1 partie du premier mélange introduit) :
(2 parties de phase vapeur évacuée)
à environ
(1 partie du premier mélange introduit) :
(15 parties de phase vapeur évacuée),
de préférence d'environ
(1 partie du premier mélange introduit) :
(3 parties de phase vapeur évacuée)
à environ
(1 partie du premier mélange introduit) :
(10 parties de phase vapeur évacuée).

13. Procédé comprenant :
(A) la mise en contact d'un premier mélange avec un second mélange dans une zone de réaction
(1) ledit premier mélange comprenant de la pyrrolidine, et
(2) ledit second mélange comprenant du 3-méthylsulfanyl-butyraldéhyde et du toluène ;
(B) la mise en réaction dans ladite zone de réaction de ladite pyrrolidine et dudit 3-méthylsulfanyl-butyraldéhyde pour l'obtention de 1-(3-méthylsulfanyl-but-1-ényl)-pyrrolidine et H₂O, ladite réaction étant effectuée dans des conditions de distillation comprenant
(1) une pression d'environ 5 000 Pa à environ 15 000 Pa, et
(2) une température d'environ 25 °C à environ 65 °C ; et
(C) l'évacuation d'une phase vapeur comprenant du toluène et H₂O et ne comprenant pratiquement pas de 3-méthylsulfanyl-butyraldéhyde,
dans lequel le rapport de
(la quantité du premier mélange introduit) : (la phase vapeur évacuée)
vaut d'environ
(1 partie du premier mélange introduit) : (3 parties de phase vapeur évacuée)
à environ
(1 partie du premier mélange introduit) : (10 parties de phase vapeur évacuée).
